# FASCICULE DE BREVET EUROPEEN

(11) **EP 1 007 220 B1**
(45) Date de publication et mention de la délivrance du brevet: **15.10.2003**
(21) Numéro de dépôt: 98942783.6
(22) Date de dépôt: 25.08.1998
(51) Int. Cl.: B05B 7/00, B05B 7/04, A61M 11/06, F23D 11/10, B01F 3/08, A61L 9/14

(54) **TETE ET APPAREIL DE NEBULISATION**
VERNEBELUNGSKOPF- UND VORRICHTUNG
SPRAYING ATTACHMENT AND APPLIANCE

(30) Priorité: 25.08.1997 FR 9710598
(43) Date de publication de la demande: 14.06.2000
(73) Titulaire: Prolitec S.A., F-34470 Perols (FR)
(72) Inventeur: BENALIKHOUDJA, Karim, F-26400 Vaunavey la Rochette (FR)
(74) Mandataire: Ravina, Bernard
(86) Numéro de dépôt international: FR9801850
(87) Numéro de publication internationale: WO99010104

(56) Documents cités:
- EP-A- 0 608 176
- GB-A- 552 689
- US-A- 2 869 188
- US-A- 3 077 307
- US-A- 4 657 007

## Description

La présente invention se rapporte à une tête de nébulisation de liquides de différentes viscosités, apte à produire des particules liquides, de dimensions inférieures à 1 µm et à les diffuser en vue de la formation d'un brouillard ou d'un aérosol.

La présente invention se rapporte également à l'appareil de nébulisation équipé d'une telle tête.

Les appareils nébuliseurs sont destinés à produire un brouillard de fines particules en vue de la purification de l'atmosphère d'un local donné comme un local ouvert au public. Dans cette application, le brouillard formé peut présenter des propriétés de désinfection, de désodorisation, de même la substance diffusée peut être une substance odorante en vue de parfumer l'atmosphère.

On peut également nébuliser des substances présentant des propriétés curatives. Ainsi, il est connu d'utiliser ce genre d'appareil tant dans le domaine médical que vétérinaire en vue notamment du traitement des voies respiratoires, de la vaccination des animaux et de l'aseptisation des locaux d'élevages.

On peut aussi utiliser de tels appareils pour la formation sous forme d'un brouillard, d'un mélange d'un air comburant avec un combustible pour l'alimentation de chaudières ou bien de moteurs thermiques.

D'autres domaines d'applications de ce genre d'appareil sont encore connus.

Quel que soit le domaine d'application de l'appareil, les résultats et les rendements sont d'autant meilleurs que le brouillard est formé de particules de taille inférieure au micron, que la taille des particules est uniforme et que le brouillard présente une aptitude naturelle à se diffuser et se répartir uniformément dans le volume dans lequel il est introduit.

Les appareils connus ne permettent pas de parvenir aux buts sus indiqués.

En effet, il a été observé avec de tels appareils, une taille de particules non constante ainsi qu'à la longue un rejet des grosses particules, ce qui se traduit par une perte d'un liquide qui peut être très coûteux par condensation sur les surfaces.

Pour éviter le rejet des grosses particules, certains nébuliseurs requièrent en sortie des formes géométriques particutières pour piéger les grosses particules interdisant toute sortie axiale du brouillard, limitant ainsi les possibilités d'intégration de ce type d'appareil dans des ensembles de transport et de distribution du brouillard formé.

De plus, les particules formées avec les appareils de l'art antérieur sont de taille trop importante pour que le brouillard ou l'aérosol puisse être transporté, sans condensation, sur des distances importantes, par exemple, dans un réseau d'air conditionné ou dans des éléments tubulaires de section interne de l'ordre demi-centimètre. ces éléments tubulaires pouvant être utilisés pour transporter le brouillard ou l'aérosol formé vers des volumes distants de plusieurs dizaines de mètres de l'appareil de nébulisation.

Avec la plupart des appareils connus, il est difficile de nébuliser avec des résultats acceptables. des liquides d'une gamme étendue de viscosité. En fait ces appareils ne fonctionnent correctement que pour une plage de viscosité déterminée qui de surcroît s'avère très étroite. En outre, ce genre d'appareil n'offre pas la possibilité d'augmenter ou de diminuer le débit nominal sans altération du résultat.

Enfin les appareils prévus pour la formation de particules de taille inférieure au micron fonctionnent avec des pressions d'air très élevées.

Une tête de nébulisation selon le préambule de la revendication 1 est connue du document EP-A-0 608 176.

La présente invention a pour objet de résoudre les problèmes sus évoqués par la mise en oeuvre d'une tête et d'un appareil de nébulisation aptes à produire des particules de taille inférieure à 1 µm et à délivrer un brouillard ou un aérosol constitué de particules de tailles uniformes constantes dans le temps qui puisse être transportés sur des distances relativement importantes sans effet notable de condensation dans la conduite de transport.

Un autre but de la présente invention est la mise en oeuvre d'une tête et d'un appareil de nébulisation qui soient aptes à nébuliser avec des résultats équivalents, des liquides d'une gamme de viscosité très étendue et ce, sous différents débits.

Enfin un dernier but de la présente invention, est la mise en oeuvre d'une tête et d'un appareil de nébulisation, aptes à former des particules de tailles inférieures au micron sous des pressions d'air faibles comprise dans une fourchette de 0,5 à huit bars.

Une tête de nébulisation selon l'invention est définie dans la revendication 1.

Selon une autre caractéristique de l'invention l'introduction de liquide est constituée par au moins un perçage cylindrique radial terminé par un second ajutage formé et dans la chambre de mélange et de fractionnement et dans le premier perçage (8), l'axe géométrique longitudinal de symétrie de la zone du second perçage, située immédiatement en amont du second ajutage est contenu dans le plan géométrique contenant le premier ajutage lequel est délimité par une arête vive formée par l'intersection de l'épanouissement avec la zone amont du premier perçage,
- l'axe longitudinal de symétrie de la zone du second perçage située immédiatement en amont du second ajutage est sécant et perpendiculaire à l'axe de symétrie de l'épanouissement.

Grâce à ces dispositions, on obtient à la sortie de la tête un jet conique constitué de particules fines de taille inférieure au micron, en nombre important et de particules plus grosses donc plus lourdes en nombre réduit.

Les caractéristiques de la tête font que l'on obtient un mélange non homogène de ces particules ; les particules les plus lourdes se situant dans la zone périphérique du jet conique alors que les particules les plus fines donc les plus légères se situent dans la zone centrale.

Ainsi, déjà à la sortie de la tête de nébulisation et ce grâce aux caractéristiques de ladite tête, est réalisée la séparation entre les grosses particules à extraire du brouillard et les fines particules devant former ce brouillard.

Pour améliorer encore ce résultat, l'épanouissement s'étend régulièrement depuis le premier ajutage jusqu'à l'ajutage de sortie, c'est-à-dire, selon toute la longueur de la chambre de mélange et de fractionnement et se présente préférentiellement sous la forme d'un tronc de cône dont l'angle au sommet est compris dans une fourchette de dix à quatre vingt dix degrés; d'excellents résultats ayant été obtenus avec un angle au sommet égal à soixante degrés. Il va de soi que ces valeurs ne sont données qu'à titre indicatif, l'angle au sommet pourra être inférieur à dix degrés ou supérieur à quatre vingt dix degrés.

La longueur de la chambre pourra être limitée à quelques millimètres, par exemple à six millimètres, cette longueur étant suffisante pour créer une zone de turbulence suffisamment importante pour assurer la séparation entre les particules.

Il y a lieu de noter que les turbulences se créent essentiellement au niveau des deux ajutages d'introduction du liquide et du gaz comprimé, en raison de l'irrégularité de surface présente à ce niveau et due à l'arête vive délimitant l'ajutage d'introduction du gaz comprimé.

Un autre facteur participant à la création de turbulences est le déséquilibre du jet formé, c'est-à-dire, son déport par rapport à l'axe de symétrie de l'épanouissement, ce déport étant dû essentiellement à la présence d'un unique ajutage d'introduction du liquide.

Pour améliorer le débit tout en conservant ce déséquilibre, l'invention selon un autre de ses aspects prévoit plusieurs seconds ajutages répartis de manière irrégulière autour de l'axe de symétrie de la chambre de mélange et de fractionnement.

En variante pourra être prévue une répartition régulière mais avec des ajutages de différents diamètres.

Selon une autre caractéristique de l'invention, la zone du premier perçage, située immédiatement en amont du premier ajutage et la zone du second perçage, située immédiatement en amont du second ajutage, sont toutes deux cylindriques. De plus la zone du premier perçage. située immédiatement en amont du premier ajutage présente un diamètre plus faible que le diamètre de la zone du second perçage, située immédiatement en amont du second ajutage. A titre d'exemple purement indicatif, ces zones présentent respectivement un diamètre sensiblement égal à 400 µm et à 800 µm. Il va de soi que ces valeurs ne sont données qu'à titre d'exemple purement indicatif, les valeurs respectives des diamètres de ces deux zones pourront être inférieures ou supérieures aux valeurs indiquées.

Par des dimensions relativement courantes dans le domaine de la mécanique de précision, il est donc possible de réaliser des ajutages aptes à créer des particules inférieures à 1 µm et ce avec les tolérances de fabrication courantes dans ce domaine.

Ceci tient au fait qu'il n'est pas recherché, essentiellement dans un souci d'efficacité et de simplification de réalisation, à produire en sortie de tête de nébulisation, des particules, toutes de tailles inférieures au micron mais au contraire à produire aléatoirement des particules fines et des particules grosses; la seule exigence étant que les particules fines qui présentent des dimensions inférieures au micron soient en nombre suffisant pour former un brouillard suffisamment dense pour pouvoir être utilisé.

Pour réduire fortement le nombre de grosses particules dans le jet émis par la tête de nébulisation, selon un autre aspect de l'invention, au-dessus de l'ajutage de sortie, à écartement de ce dernier, est monté un déflecteur plan, ce déflecteur étant normal à l'axe de symétrie de la chambre et centré par rapport à ce dernier, ledit déflecteur présentant des largeur et longueur supérieure au diamètre de l'ajutage de sortie et étant notamment destiné à rabattre latéralement le jet vers l'arête formant l'ajutage de sortie de la chambre de mélange. Cette disposition permet de réaliser à ce niveau c'est à dire sur l'ajutage de sortie un fractionnement des grosses particules. Pour renforcer ce résultat l'ajutage de sortie est formé par une arête vive. Accessoirement le déflecteur est destiné à capter et dévier les grosses particules.

La taille des particules formées dépend étroitement de l'écart entre le déflecteur et l'ajutage de sortie. A titre d'exemple, cet écart sera supérieur à 5 µ m et pourra être compris entre 100 µ m et 5 mm. Selon le cas, l'écart entre le déflecteur et l'ajutage de sortie pourra être fixe ou bien ajustable afin de régler la taille des particules émises et d'adapter la tête de pulvérisation à la viscosité du liquide à nébuliser. L'écart entre le déflecteur et l'ajutage de sortie détermine le débit de la nébulisation. De manière inattendue à écart faible correspond un débit important.
D'excellents résultats ont été obtenus avec un écartement de 150 µ m.

Enfin la tête de nébulisation, selon une autre forme de réalisation, pourra être pourvue d'un perçage prévu pour l'aspiration des écoulements résiduels, ce second perçage débouchant d'une part dans la chambre de mélange et de fractionnement et d'autre part sur l'une des faces du corps de la tête où il forme à ce niveau un orifice d'aspiration des écoulements résiduels de liquide formés par les grosses particules.

Les différentes caractéristiques de la tête de nébulisation telles qu'elles viennent d'être exposées permettent la nébulisation avec des résultats identiques ou pour le moins comparables de liquides de viscosité très éloignée tels que des solutions aqueuses et des huiles et ce sous une gamme de pression de gaz et donc de débit, très étendue par exemple, en ce qui concerne, les pressions de 0,5 bars à 8 bars.
Par ailleurs, la longueur réduite de la chambre de fractionnement et de mélange de la tête de nébulisation permet d'apporter une réponse efficace aux contraintes dimensionnelles posées par l'implantation dans des appareils de nébulisation de tailles réduites.

L'appareil de nébulisation conforme à l'invention se caractérise essentiellement en ce qu'il est équipé d'au moins une tête de nébulisation telle que précédemment décrite

Cet appareil, selon une autre caractéristique. comprend un corps creux à la base duquel et dans lequel est monté au moins une tête de nébulisation en relation d'une part avec une source de gaz comprimé par l'intermédiaire d'une conduite et d'autre part avec un réservoir de liquide à nébuliser, monté sous le corps, la tête de nébulisation, par son ajutage de sortie est en communication avec une chambre de détente pratiquée dans le corps de l'appareil, coaxialement à la chambre de mélange et de fractionnement de la tête de nébulisation, la face interne de ladite chambre recevant les grosses particules émises par le jet et captant ces dernières, lesquelles sous l'effet de leur poids s'écoulent sur ladite face, vers le réservoir.

Lorsque la tête est équipée du déflecteur, le jet nébulisé est amené à tournoyer dans la chambre de détente et ce en raison de la surface en pente de ladite tête dont le rôle essentiel est d'accompagner et diriger ce jet.

Le jet de particules émis par la tête de nébulisation, en ce qui concerne la zone périphérique de ce dernier, c'est-à-dire celle formée par les grosses particules, est orienté vers la face interne de la chambre de détente de façon que les grosses particules soient captées par cette dernière.

Selon une autre caractéristique de l'invention, la chambre de détente à l'opposée de la tête de nébulisation, est limitée par une paroi transversale perpendiculaire à l'axe géométrique de la chambre de fractionnement, cette paroi en son centre comportant d'un perçage traversant de sortie de la nébulisation. La hauteur de cette paroi transversale par rapport à la tête de pulvérisation ainsi que le diamètre du perçage traversant pourront varier en fonction par exemple des caractéristiques physiques des liquides à nébuliser.

Selon encore une autre caractéristique de l'invention, la face externe à la chambre de nébulisation, de la paroi transversale, est convexe et ladite paroi en périphérie comporte des échancrures. Cette disposition permet l'écoulement vers la chambre de détente de l'éventuel condensat formé au dessus ou sur la paroi transversale.

La face interne de la chambre de détente pourra être lisse, ou bien être dotée d'une ou plusieurs saillies ou creux.

Cependant, que la configuration de la face interne de la chambre soit lisse ou avec des reliefs, elle entraîne une perte de charge importante.

De cette perte de charge, il en résulte une baisse de la vitesse de progression du brouillard de micro-particules dans l'appareil, un risque de dépôt et de condensation, dans le temps, de ces dernières sur la face interne du corps et par voie de conséquence, un risque d'entraînement de particules de tailles plus importantes dans le jet de micro-particules.

Pour pallier à cet inconvénient, selon une autre caractéristique de l'invention, la chambre de détente est prolongée par au moins une zone d'accélération du mouvement du brouillard de particules afin d'augmenter la vitesse de ce dernier et éviter tout dépôt de particules fines, ladite zone étant en relation avec un conduit de sortie du brouillard ou de l'aérosol formé.

La vitesse des particules dans la zone d'accélération est suffisamment importante pour éviter d'être captée et de se condenser contre les parois du corps creux.

Dans la forme préférée de réalisation, la zone d'accélération du mouvement du brouillard est en relation avec la chambre de détente par un passage de section plus faible que la section de ladite chambre, ce passage de section plus faible pouvant être constitué, lorsque la chambre de détente est limitée par la paroi transversale, par le perçage traversant que comporte cette dernière.

Ce passage de section réduite est propice en lui-même, en sortie, à délivrer un jet selon une vitesse élevée.

Selon une autre forme de réalisation de l'invention, la chambre de détente, à l'opposé de la tête de nébulisation comporte une paroi d'obturation et la paroi du réservoir, au-dessus du niveau maximal de liquide, est équipée d'un embout de sortie du brouillard ou de l'aérosol. Cette forme de réalisation, qui ne prévoit plus de zone d'accélération supérieure conduit à réaliser un appareil particulièrement compact.

Selon une autre caractéristique de l'invention le corps de l'appareil, entre le réservoir et la chambre de détente, est doté d'une cloison transversale de séparation portant la tête de nébulisation. Cette cloison, pour un appareil doté d'une zone d'accélération supérieure. assurera l'étanchéité entre la chambre de détente et la zone du réservoir située au dessus du niveau de liquide. Cette disposition évite que sous l'effet de la pression, le brouillard de particules soit ramené vers le bas et dans le réservoir, ce qui affaiblirait de manière conséquente le rendement de l'appareil.

Pour un appareil dont la sortie du brouillard ou de l'aérosol est ménagée dans la paroi du réservoir, la cloison de séparation sera dotée d'une ouverture de communication de la chambre de détente avec le volume du réservoir situé au-dessus du niveau de liquide.

Selon une autre caractéristique de l'invention, l'appareil de nébulisation est doté d'un moyen d'évacuation vers le réservoir des écoulements résiduels de liquide formés par les grosses particules dans la chambre de détente. Cette disposition évite l'engorgement de la chambre de détente par ces écoulements.

Selon une autre caractéristique de l'invention, le moyen d'évacuation des écoulements résiduels vers le réservoir traverse de part en part la cloison de séparation, ce moyen, par son extrémité inférieure, étant situé en dessous du niveau minimal de liquide dans le réservoir. Cette disposition qui prévoit un moyen d'évacuation interne à l'appareil conduit aussi à une meilleure compacité de ce dernier.

Pour un appareil dont la chambre de détente est séparée de manière étanche de la zone du réservoir située au-dessus du niveau de liquide, le moyen d'évacuation vers le réservoir, des écoulements résiduels de liquide formés par les grosses particules, est constitué par au moins un tube engagé dans un perçage pratiqué dans la cloison de séparation transversale, ladite cloison et ledit tube assurant de plus la séparation étanche entre le volume du réservoir situé au dessus du niveau de liquide et la chambre de détente.

Pour un appareil dont la chambre de détente est en communication avec la zone du réservoir située au-dessus du niveau de liquide, le moyen d'évacuation est constitué par une lame engagée dans l'ouverture de communication.

Pour un appareil équipé d'une cloison transversale assurant la séparation étanche entre la chambre de détente et le réservoir de liquide à nébuliser, il est aussi possible d'éviter l'engorgement de ladite chambre par accumulation des écoulements résiduels en utilisant une tête de nébulisation équipée d'un perçage d'aspiration débouchant sur l'une des faces du corps de la tête.

D'autres buts, avantages et caractéristiques de l'invention apparaîtront à la lecture de la description de formes préférées de réalisation données à titre d'exemple non limitatifs en se référant aux dessins annexés en lesquels :
- la figure 1 est une vue en coupe longitudinale d'une tête de nébulisation selon une première forme de réalisation,
- la figure 2 est une vue d'une tête de nébulisation selon une seconde forme de réalisation,
- la figure 3 est une vue en coupe d'une tête selon une autre forme de réalisation,
- la figure 4 est une vue en coupe d'une tête de nébulisation avec perçage radial pour l'aspiration des écoulements résiduels,
- la figure 4a est une vue en coupe d'une tête de nébulisation avec perçage radial pour l'aspiration des écoulements résiduels selon une seconde forme de réalisation.
- la figure 5 est une vue en coupe longitudinale d'un appareil selon une première forme de réalisation.
- la figure 6 est une vue en coupe longitudinale d'un appareil de nébulisation selon une seconde forme de réalisation,
- la figure 7 est une vue en coupe d'un appareil selon une troisième forme de réalisation,
- la figure 8 est une vue en coupe d'un appareil selon une quatrième forme de réalisation,
- la figure 9 est une vue en coupe d'un appareil selon une cinquième forme de réalisation,
- la figure 10 est une vue en coupe longitudinale d'un appareil selon une sixième forme de réalisation.
- la figure 11 montre vu de dessus. un agencement de têtes de nébulisation selon une première forme de réalisation,
- la figure 12 montre, vu de dessus, un agencement de têtes de nébulisation selon une seconde forme de réalisation.

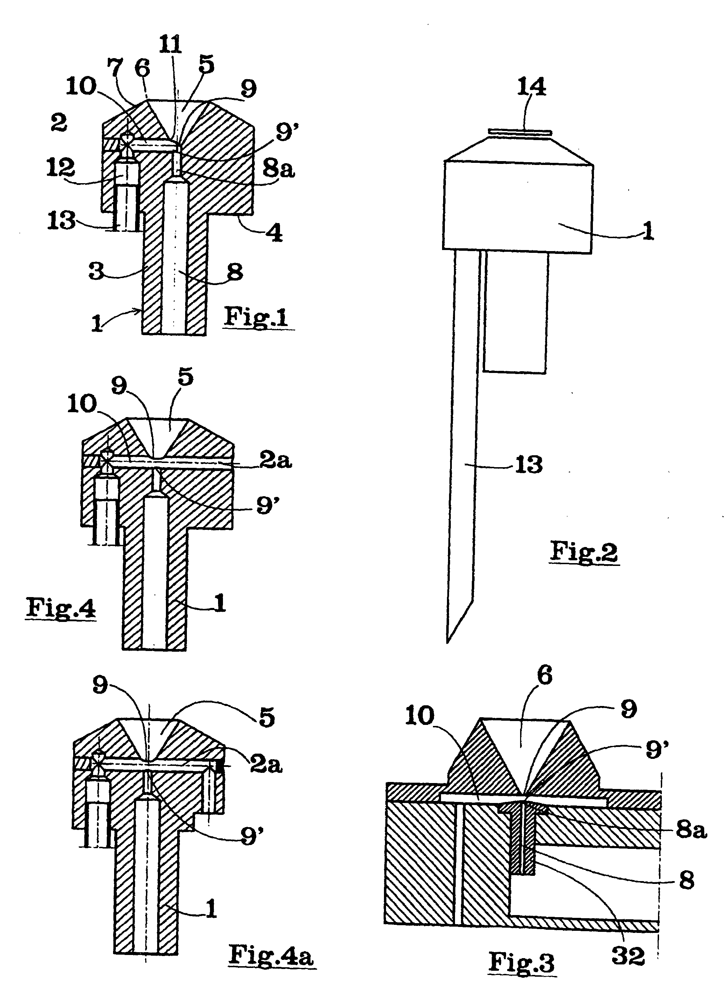

En figure 1 est représentée une tête de nébulisation 1 selon une première forme de réalisation de l'invention.

Cette tête comprend un corps formé de deux formes cylindriques 2, 3 axialement alignées, de différents diamètres, séparées l'une de l'autre par une surface épaulée 4, le diamètre de la forme cylindrique 3 étant plus faible que le diamètre de la forme cylindrique 2. Selon l'axe longitudinal du corps, dans la forme cylindrique 2, depuis la face avant de cette dernière, est pratiquée à l'aide de tous moyens connus, une chambre de mélange et de fractionnement 5 en forme de tronc de cône inversé, la grande base du tronc de cône étant située dans la face avant et étant délimitée par une arête vive circulaire formant ainsi l'ajutage de sortie 6 de la tête 1. De préférence, l'ajutage de sortie 6 est contenu dans un plan normal à l'axe de symétrie de la chambre 5. Autour de l'ajutage de sortie 6, le corps de la tête présente une surface en pente 7 formant un tronc de cône. Cette surface en pente, en permettant l'écoulement vers le bas des grosses particules qui s'y déposent, évite que ces dernières soient entraînées dans le jet. Dans l'axe de la chambre de mélange et de fractionnement 5, la seconde forme cylindrique 3 est traversée de part en part par un perçage 8, ou premier perçage. Ce perçage débouche dans la chambre de mélange et de fractionnement 5 pour y former, suivant la petite base de la forme en tronc de cône de ladite chambre, un premier ajutage circulaire 9 délimité par une arête vive et contenu dans un plan géométrique normal à l'axe longitudinal de symétrie de la chambre 5. Le perçage traversant 8 se termine immédiatement en amont de l'ajutage 9 par,une zone 8a de plus faible diamètre, de l'ordre par exemple de 400 µ m, le diamètre du premier ajutage 9 étant égal à cette valeur. La longueur de la zone 8a détermine la plage des pressions de fonctionnement à savoir de la pression du fluide gazeux en amont du perçage 8. Ainsi avec une longueur de 5 à 6 mm la plage de fonctionnement est comprise entre 0,5 bars et 3,5 bars ; avec une longueur de 2 mm elle est comprise entre 2 et 6 bars. En dessous des valeurs minimales l'effet venturi ne se produit pas. Au dessus des valeurs maximales, la nébulisation, ne se produit pas non plus ;une partie du fluide gazeux s'engouffre dans l'introduction 10 de liquide et repousse le liquide vers le réservoir.

La seconde forme cylindrique 3 forme une canule et est destinée à être raccordée par une conduite à une source de distribution de gaz sous pression, ce gaz pouvant être de l'air.

Selon le plan géométrique du premier ajutage 9, la première forme cylindrique est pourvue d'un perçage radial cylindrique 10 dont l'axe longitudinal de symétrie est radial au premier ajutage. Ce second perçage débouche dans la chambre de mélange et dans la zone 8a du premier perçage et forme à ce niveau un second ajutage 11 s'étendant de part et d'autre du plan géométrique du premier et un troisième ajutage 9'résultant de l'intersection de la surface cylindrique de ce second perçage avec la surface cylindrique du premier perçage. Immédiatement en amont de ce second ajutage 11, le perçage radial 10 présente une zone de plus faible diamètre 10a, la valeur du diamètre de cette zone étant par exemple de l'ordre de 800 µ m. A l'opposé de l'ajutage 11, le perçage radial 10 est obturé par un tampon approprié. A titre d'exemple, ce tampon sera fileté et sera engagé dans un taraudage pratiqué dans le perçage radial. Depuis la face épaulée est pratiquée dans la première forme cylindrique, parallèlement à l'axe longitudinal de cette dernière, un perçage d'alimentation 12 débouchant dans le perçage 10. Dans ce perçage 12, depuis la surface épaulée 4, est réalisé un lamage taraudé dans lequel s'engage en vissage l'extrémité filetée d'un tube d'aspiration 13 du liquide à nébuliser.

Par passage de l'air comprimé depuis l'ajutage 9' vers l'ajutage 9 est créée au niveau de l'ajutage 11, une dépression sous l'effet de laquelle, si la vitesse de l'air comprimé est suffisamment élevée, le liquide à nébuliser est aspiré dans le tube 13 ainsi que dans les perçages 12 et 10 pour parvenir à l'ajutage 9 et ensuite se fractionner et se mélanger au flux d'air dans l'épanouissement tronconique que forme la chambre 5.

Cette buse de nébulisation telle que décrite délivre en sortie un brouillard sous forme de jet conique contenant des grosses particules dans les zones périphériques et des particules de taille inférieure à 1 µ m dans les zones centrales.

En figure 2 on peut voir que la tête de nébulisation selon l'invention, est équipée d'un déflecteur 14 se présentant par exemple sous la forme d'une lame rectangulaire plane présentant des longueur et largeur supérieures au diamètre de l'ajutage de sortie. Ce déflecteur 14 est parallèle au plan de l'ajutage de sortie 6 et est maintenu de manière centrée par rapport à l'axe longitudinal de symétrie de la chambre 5. De plus, pour permettre l'ajustement de la taille des particules et la délivrance de particules de tailles adaptées à l'application envisagée, l'écart entre le déflecteur 14 et l'ajutage de sortie est réglable. A un écart faible, de l'ordre du dixième de millimètre, correspond la formation de fine particules en raison de l'accentuation des turbulences dans la chambre ce qui renforce le fractionnement, tandis qu'à un écart important de l'ordre du demi-centimètre correspond une diminution de l'importance des turbulences dans la chambre d'où un moindre fractionnement des particules.

La tête de nébulisation, telle qu'elle vient d'être décrite, peut être dotée comme on peut le voir en figure,4 d'au moins un perçage radial 2a débouchant d'une part dans la chambre de mélange et de fractionnement 5 et d'autre part sur la face cylindrique de la première forme cylindrique 2 du corps de la tête ou il forme à ce niveau un orifice d'aspiration des écoulements résiduels de liquide formés par les grosses particules. Ainsi, toute accumulation d'écoulement résiduel se trouve aspirée dans la tête pour subir de nouveau dans la chambre de fractionnement 5 une nébulisation.

En variante, telle que représentée en figure 4a, le perçage 2a présente une partie verticale pour déboucher non pas dans la face cylindrique de la première forme cylindrique du corps de la tête de nébulisation mais dans un décrochement qu'il présente en partie en partie inférieure. Selon cette forme de réalisation, l'aspiration des écoulements résiduels s'effectue depuis le décrochement.

En figure 3 on a représenté une tête selon une autre forme de réalisation. On peut voir que l'introduction 10 de liquide s'étend entre et autour des ajutages 9 et 9' ces derniers étant écartés de préférence d'une valeur de quelques dixièmes de millimètres. De préférence, sans que cela soit limitatif, les ajutages 9 et 9', toujours délimités chacun par une arête vive, présentent le même diamètre. Cette tête est constituée d'un corps en deux parties assemblées l'une à l'autre de manière démontable. La chambre 6 de mélange et de fractionnement ainsi que l'introduction 10 sont formées dans la première partie de corps tandis que la seconde partie du corps est équipée du premier perçage 8 et des conduites de raccordement du perçage 8 et de l'introduction 10 à la source de gaz sous pression et à la réserve de liquide à nébuliser. Préférentiellement l'introduction 10 est constituée par une chambre cylindrique ménagée à la base du premier corps de tête. Lors de l'assemblage des deux parties de corps de tête, la chambre cylindrique est positionnée en regard d'une surface plane de jonction que comporte la seconde partie du corps de tête. Toujours selon la forme préférée de réalisation, le perçage 8 est formé dans une buse amovible 32 introduite dans un logement de la seconde partie de corps, formé de manière traversante depuis la surface plane de jonction vers un perçage borgne raccordé par tous moyens approprié à la source de gaz comprimé. Cette buse amovible comme on peut le voir en figure 3 comporte une tête cylindrique logée dans un lamage du logement. La tête de la buse comporte autour de l'ajutage 9' une surface en pente tronconique formant saillie sur la surface de jonction. On peut remarquer que la surface cylindrique de la tête est totalement logée dans le lamage, seule forme saillie la surface en pente. Cette disposition permet d'accompagner le liquide à nébuliser vers le flux d'air comprimé passant de l'ajutage 9' vers l'ajutage 9.

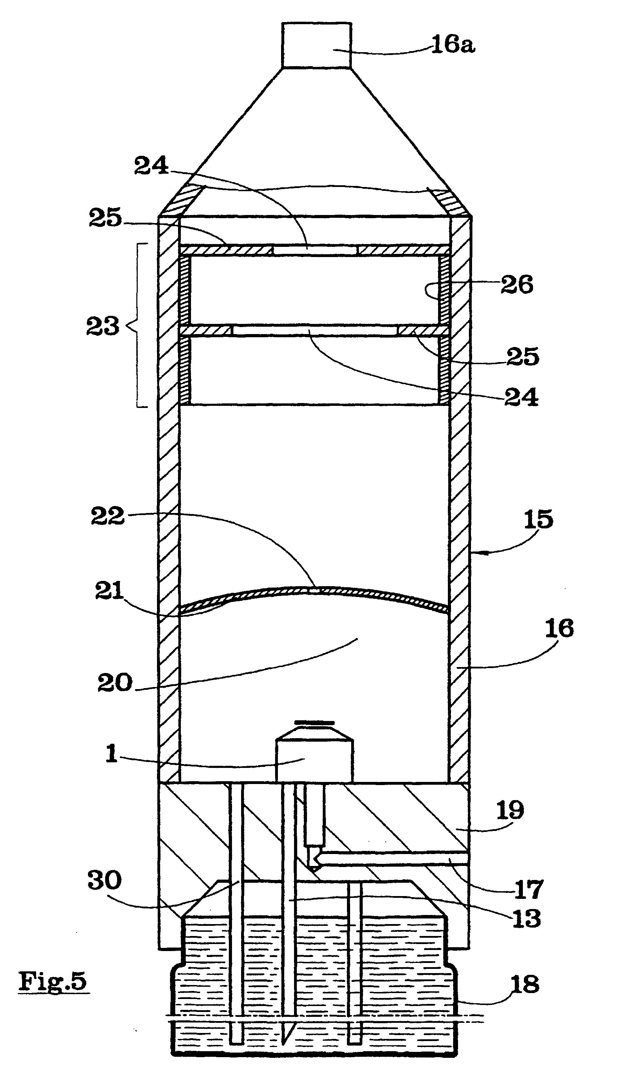

En figure 5 est représenté un appareil de nébulisation 15 selon une première forme de réalisation.
Cet appareil comporte au moins une tête de nébulisation 1 selon l'une ou l'autre forme de réalisation et comprend un corps 16 creux à la base duquel est montée au moins une tête de nébulisation 1 alimentée en gaz comprimé, par exemple de l'air, par une conduite 17 connectée à une source de gaz comprimé. La tête est alimentée en liquide à nébuliser par le tube 13 d'aspiration, ce tube plongeant dans un réservoir de liquide 18 monté sous le corps 16 et séparé de ce dernier par une cloison transversale de séparation 19 portant la tête de nébulisation 1. Le jet de particules produit par la tête 1 est admis dans une chambre de détente 20 formée dans le corps. au-dessus de la cloison transversale de séparation 19. A l'opposé de la tête de nébulisation, le corps de l'appareil est doté d'un perçage traversant, taraudé dans lequel est engagé l'embout fileté d'un conduit de sortie 16a du brouillard de particules.

Dans la forme préférée de réalisation, la chambre de détente 20 est de forme cylindrique mais en variante la forme de ladite chambre pourra être parallélépipédique. La chambre de détente 20 et la chambre de mélange et de fractionnement 5 de la tête de nébulisation sont coaxiales l'une à l'autre. Toujours selon la forme préférée de réalisation, la cloison de séparation 19 est prévue avec un alésage traversant et un alésage borgne parallèles l'un à l'autre, l'alésage borgne étant pratiqué dans la paroi 19 depuis la face tournée vers la chambre de détente. Dans ces alésages sont engagés respectivement avec interposition de joints d'étanchéité toriques. d'une part, le tube d'aspiration 13 et d'autre part, la seconde forme cylindrique 3 du corps de la tête. L'alésage borgne est en communication avec un perçage radial pratiqué dans la paroi transversale depuis la face externe du corps de l'appareil. Ce perçage radial est en communication avec une conduite (non représentée) raccordée à une source d'air comprimée pour assurer l'alimentation de la tête de nébulisation en air comprimé.

La chambre de détente 20 est préférentiellement définie par le volume interne du corps de l'appareil.

De préférence la chambre de détente 20 à l'opposée de la tête de nébulisation 1 est limitée par une paroi transversale 21 perpendiculaire à l'axe géométrique de la chambre de fractionnement de ladite tête, cette paroi en son centre comportant un orifice traversant 22 de sortie de la nébulisation.

Avantageusement la face externe à la chambre de nébulisation, de la paroi transversale 21 est convexe et comporte en périphérie des échancrures. Cette disposition permet l'écoulement gravitaire vers la chambre de détente et vers le réservoir des éventuels condensats de liquides formés au dessus de la paroi ou bien sur la face convexe.

Dans la forme de réalisation objet de la figure 5, la chambre de détente 20 est prolongée axialement vers le haut par une zone 23 d'accélération du mouvement du brouillard de particules afin d'augmenter la vitesse de ce dernier et éviter tout dépôt de particules sur les faces du volume interne du corps de l'appareil.

Selon la forme préférée de réalisation, la zone d'accélération 23 est en relation avec la chambre de détente 20 par un passage 24 de plus faible section que la section de ladite chambre. Ce passage 24 est constitué par exemple par un perçage traversant formé dans une cloison de séparation 25 s'étendant transversalement dans le corps creux et maintenue fixement dans le corps contre la rive supérieure du fourreau tubulaire 20a ou à distance de cette dernière. Ce perçage traversant 24 pourra être formé par une lumière oblongue mais toute autre forme de perçage pourra convenir. Avantageusement, la zone d'accélération comprend au moins deux cloisons de séparation 25 avec lumières traversantes, montées à écartement l'une de l'autre, la lumière traversante de l'une étant décalée angulairement de quatre vingt dix degrés par rapport à la lumière traversante de l'autre afin que le jet, entre les deux cloisons, subisse une rotation selon un quart de tour ce qui accroit la vitesse tangentielle des particules et diminue le risque d'agglomération de ces dernières sur les faces du volume interne du corps de l'appareil. Les cloisons de séparation 25 pourront être maintenues à distance l'une de l'autre d'une part et à distance du fourreau 20a d'autre part par un jeu de plusieurs entretoises tubulaires 26 montées dans le volume interne du corps de l'appareil.

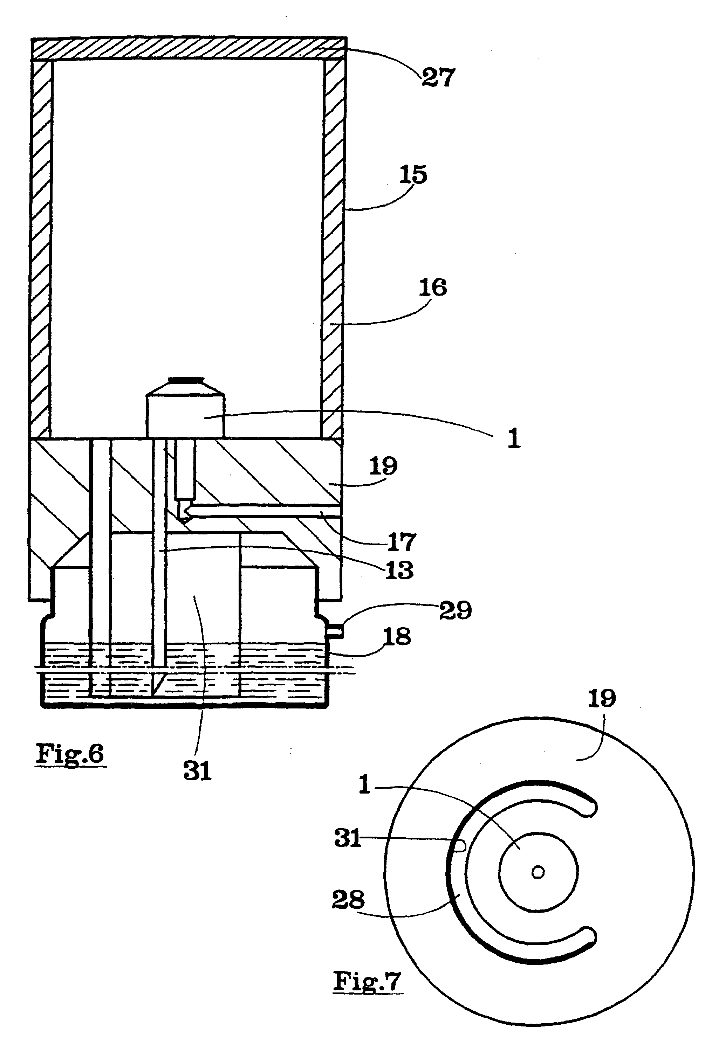

L'appareil de nébulisation selon la figure 6 n'est plus doté de zone d'accélération supérieure disposée au-dessus de la chambre de détente 20 et cette dernière, à l'opposé de la tête de nébulisation 1, comporte une paroi d'obturation 27 fixée de manière connue en soi à la rive supérieure du fourreau tubulaire 20a. Selon cette variante, la cloison transversale de séparation 19 est pourvue d'une ouverture 28 de communication de la chambre de détente 20 avec le volume du réservoir situé au-dessus du niveau de liquide et la paroi du réservoir 18, au-dessus du niveau maximal de liquide, est équipée d'un perçage taraudé dans lequel est engagé en vissage l'embout fileté d'un conduit 29 de sortie du brouillard ou de l'aérosol formé. L'ouverture de communication 28 sera ménagée dans la cloison 19 autour du corps de la tête de nébulisation 1 selon un arc de circonférence de cercle.

De préférence, l'appareil de nébulisation, selon l'une ou l'autre forme de réalisation telles que décrites, est équipé d'un moyen d'évacuation vers le réservoir, des écoulements résiduels de liquide formés par les grosses particules dans la chambre de détente. De préférence, ce moyen traverse de part en part la cloison de séparation et vient par son extrémité inférieure en dessous du niveau minimal de liquide dans le réservoir.

Pour l'appareil selon la figure 5 le moyen d'évacuation consistera en au moins un tube vertical 30 engagé par son extrémité supérieure dans un perçage traversant pratiqué dans la cloison de séparation 19, ladite cloison 19 et ledit tube 30 assurant de plus la séparation étanche entre le volume du réservoir situé au dessus du niveau de liquide et la chambre de détente. Par son extrémité inférieure, le tube 30 vient au-dessous du niveau minimal de liquide dans le réservoir 18. Pour une évacuation complète des écoulements résiduels, pourront être prévus dans la cloison 19, autour du corps de la tête de nébulisation, plusieurs perçages traversants associés chacun à un tube d'évacuation qui leur est propre.

Pour l'appareil selon la figure 6, le moyen d'évacuation sera constitué par une lame verticale de faible épaisseur, engagée par son extrémité supérieure dans l'ouverture de communication et plaquée contre une des lèvres longitudinale de cette dernière en la recouvrant en totalité. Le liquide résiduel en glissant sur cette lame est conduit par ruissellement vers le réservoir 18 et le liquide contenu dans ce dernier.

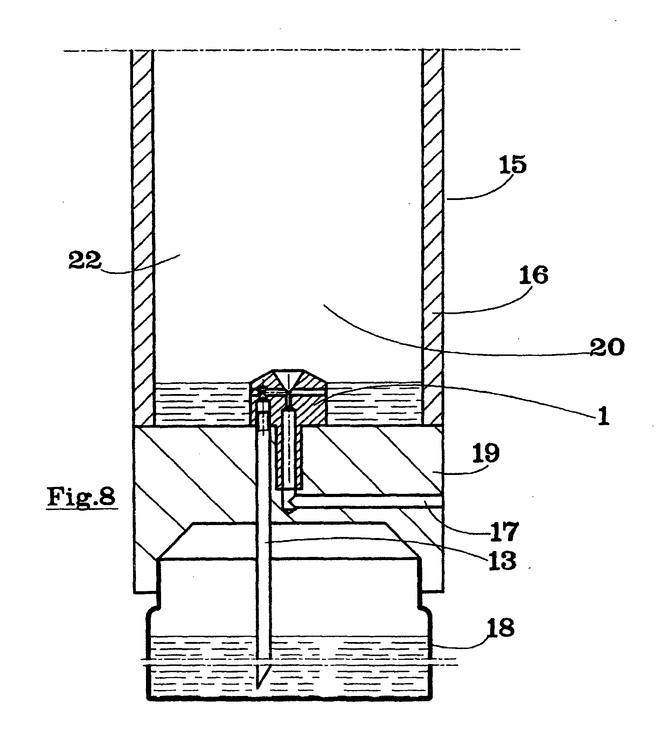

En figure 8 est représenté de manière partielle un appareil de nébulisation dont la tête de nébulisation est équipée d'un perçage 2a d'aspiration du liquide résiduel. Ce perçage peut déboucher dans la face cylindrique de la première forme cylindrique du corps de tête ou bien en variante, comme illustrée en figure 4a, présenter une partie verticale pour déboucher dans un décrochement ménagé dans ladite première forme cylindrique. Selon l'une ou l'autre de ces deux formes de réalisation, l'appareil est équipé d'une cloison 19 assurant une séparation étanche entre le réservoir dans son ensemble et la chambre de détente. L'appareil selon cette forme de réalisation n'est pas doté de moyen d'évacuation vers le réservoir. des écoulements résiduels ce qui simplifie sa réalisation.

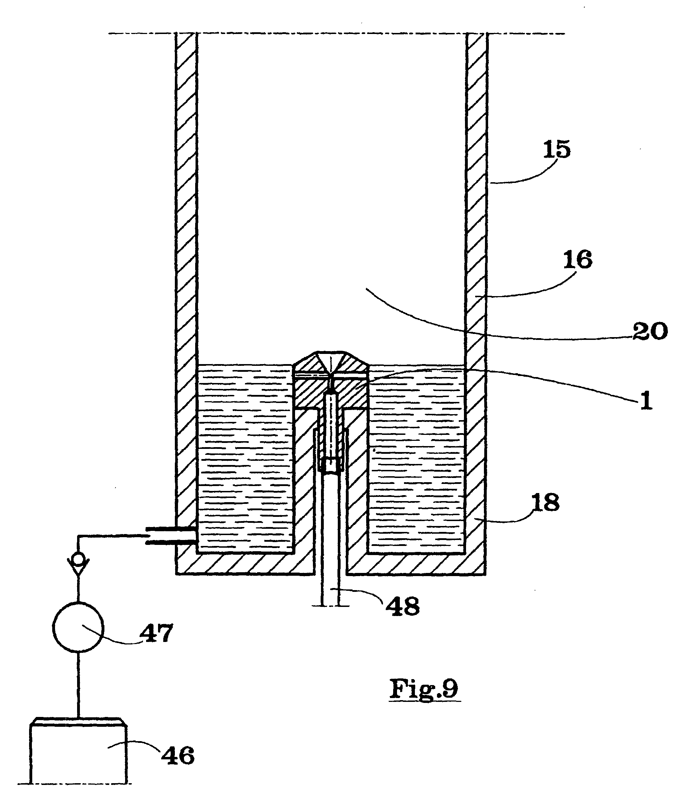

En figure 9 est représenté un appareil de nébulisation exempt de cloison de séparation 19. Dans cette forme de réalisation, le volume interne du réservoir est directement en communication avec la chambre de détente 20 et comporte en saillie sur son fond une proéminence cylindrique portant la tête de nébulisation 1. Cette dernière ne comporte pas de perçage d'alimentation et le perçage radial 12 débouche maintenant sur la face cylindrique de la première forme cylindrique de la tête 1 pour former à ce niveau un ajutage d'aspiration situé au dessous du niveau de liquide dans le réservoir. Cette tête pourra aussi être dotée d'un perçage d'aspiration du liquide résiduel débouchant, soi dans la face cylindrique de la première forme cylindrique du corps de tête, soi dans un décrochement formé en partie inférieure. De plus le perçage 12 pourra comporter une partie verticale et déboucher non pas dans la surface cylindrique de la première forme cylindrique du corps de tête mais dans un décrochement formé en partie inférieure du corps de la première forme cylindrique du corps de tête.

L'appareil est associé à des moyens pour maintenir constant le niveau de liquide dans le réservoir, ces moyens pouvant être constitués par une capacité de stockage 46 de liquide à nébuliser. externe à l'appareil et par une pompe 47 dont l'orifice d'aspiration est connecté, par une conduite appropriée, au volume interne de la capacité de stockage. Par l'intermédiaire d'une conduite appropriée, l'orifice de refoulement de la pompe sera connecté au volume interne du réservoir 18. Le débit de la pompe pourra être calé sur le débit de liquide nébulisé ou bien des capteurs de niveau, non représentés, associés à un circuit de pilotage de la mise en marche et de l'arrêt du fonctionnement de la pompe 47, pourront être disposés dans le réservoir. Dans cette forme de réalisation, le résiduel de liquide retombe directement dans le réservoir 18. Selon cette forme de réalisation, la tête de nébulisation est alimentée en air comprimé par une canule verticale 48.

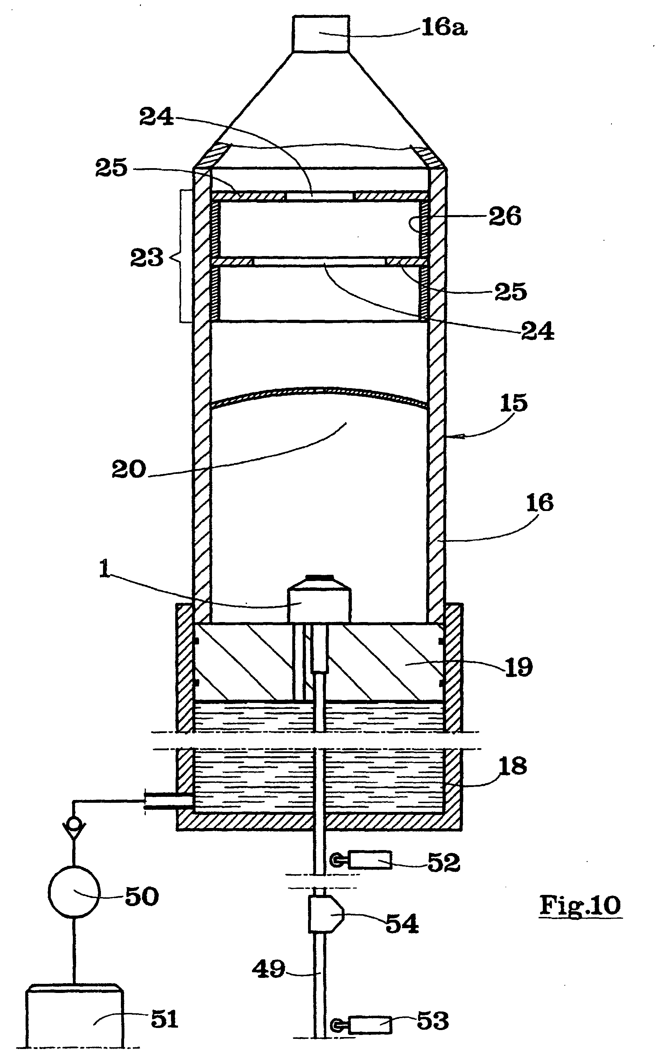

Pour éviter la présence de capteur de niveau dans le réservoir de liquide, on peut prévoir un appareil avec cloison de séparation 19, monté de manière coulissante dans un réservoir à la manière d'un piston. Dans cette forme de réalisation, telle qu'illustrée en figure 10, l'appareil 15 comporte une surface externe cylindrique et coulisse avec peu de jeu, par cette surface cylindrique externe, dans l'alésage cylindrique du réservoir 18. La partie inférieure du corps de l'appareil et plus particulièrement la cloison de séparation 19 est agencée en piston et comporte un ou des segment(s) d'étanchéité, annulaire(s). Selon cette forme de réalisation, l'alimentation en air comprimé n'est plus effectuée par un orifice radial pratiqué dans la cloison de séparation 19, mais par une canule rigide 49, en communication avec le perçage 8 du corps de la tête de nébulisation par l'intermédiaire d'un perçage axial traversant, pratiqué dans ladite cloison 19 Cette canule 49 s'étend dans le réservoir 18 selon la direction de coulissement du corps de l'appareil, et est engagée en coulissement dans un alésage pratiqué dans la paroi de fond du réservoir, la canule traversant de part en part le fond de ce réservoir. Des joints d'étanchéité annulaires sont disposés dans l'alésage pour éviter toute fuite de liquide à ce niveau. A l'extérieur du réservoir. la canule est connectée par une conduite souple à une source de distribution d'air sous pression. L'appareil selon cette forme de réalisation est porté par le liquide présent dans le réservoir 18. Ainsi le degré d'enfoncement du corps de l'appareil dans le réservoir dépend de la quantité de liquide contenu dans ce dernier. De ce fait, par mesure ou détection du degré d'enfoncement, il est possible de détecter la quantité de liquide dans le réservoir et si le niveau est trop bas de réapprovisionner de manière automatique ce dernier à l'aide d'une pompe 50 dont l'orifice d'aspiration de liquide est connecté par une conduite appropriée, au volume interne d'une réserve de liquide 51. L'orifice de refoulement de cette pompe est connecté au volume interne du réservoir 18 par l'intermédiaire d'une conduite appropriée. Cette pompe 50 sera actionnée par un moteur électrique, piloté par un circuit d'automatisme et de puissance comprenant notamment à l'extérieur du réservoir 18, deux capteurs de niveau 52, 53 distants l'un de l'autre et disposés tous deux sur le trajet d'un plot d'actionnement 54 porté par la canule. Le capteur le plus proche du réservoir ou premier capteur, délivre lorsqu'il est actionné, un signal représentatif d'un niveau maximal de liquide dans le réservoir 18, tandis que le second capteur délivre lorsqu'il est actionné un signal représentatif d'un niveau minimal de liquide. L'application de ce dernier signal sur l'entrée correspondante du circuit d'automatisme et de puissance commande la fermeture du circuit d'alimentation du moteur électrique d'actionnement de la pompe et donc l'activation de la pompe 50. La pompe aspire alors le liquide contenu dans la réserve 51 et le refoule vers le réservoir 18. Le liquide introduit repousse progressivement l'appareil 15 vers sa position de moindre enfoncement dans le réservoir, matérialisée par la position du premier capteur. Lorsque ce dernier est actionné par le plot 54 porté par la tige 49, le signal émis et appliqué sur l'entrée correspondante du circuit d'automatisme et de puissance, se traduit par l'ouverture du circuit d'alimentation du moteur électrique et donc la désactivation de la pompe.

Selon un autre mode d'exécution, le moyen pour maintenir constant le niveau de liquide dans le réservoir 18 peut être constitué par un réservoir annexe, étanche, attenant à l'appareil et pourvu en partie inférieure d'un embout de sortie connecté par une conduite à un embout d'entrée de liquide que comporte le réservoir 18 en partie inférieure.

L'appareil, dans ces différentes formes de réalisation telles que décrites, peut être équipé de plusieurs têtes 1 de nébulisation alimentées en liquide à nébuliser à partir d'un même réservoir et en gaz comprimé à partir d'une même source de pression.

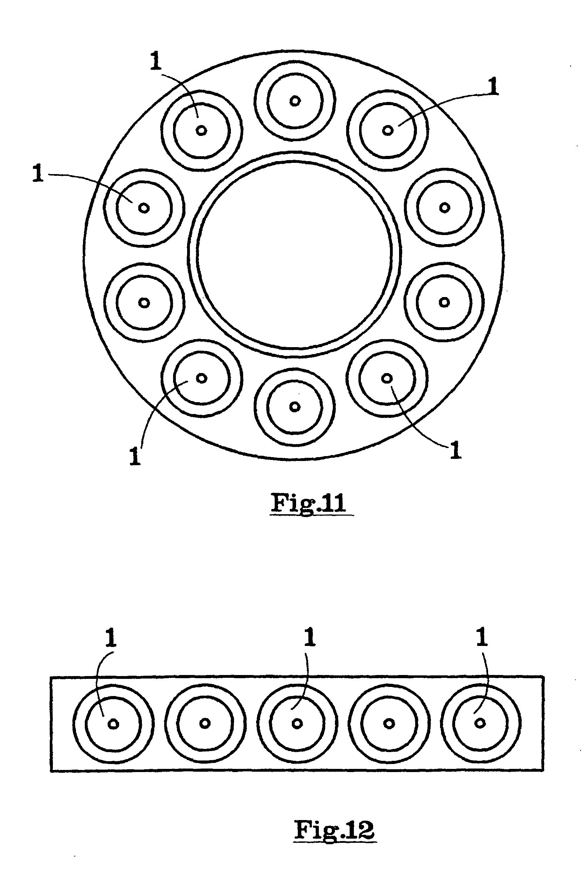

En figure 11 est représenté un premier agencement de tête. Selon cette forme de réalisation, les têtes de nébulisation 1 sont réparties à intervalles réguliers ou irréguliers selon un cercle, dont le centre par exemple sera situé sur l'axe géométrique médian vertical de la chambre de détente. Cet agencement convient plus particulièrement à une utilisation avec une chambre de détente de forme cylindrique.

En figure 12 est représenté un second agencement de tête de nébulisation. Selon cette autre forme de réalisation, les têtes de nébulisation sont réparties à intervalles réguliers ou irréguliers selon une droite. Cet agencement peut être utilisé avec une chambre de détente de section droite rectangulaire, pour une meilleure efficacité, l'alignement formé par les têtes de nébulisation se développera selon le sens de la longueur de ladite section droite. L'angle d'inclinaison de chaque tête par rapport à une référence pourra être fixe ou bien variable.

## Revendications

1. Tête de nébulisation (1) comportant un corps dans lequel est formé une chambre ouverte (5) de mélange et de fractionnement pourvue d'un ajutage de sortie (6) et, de manière opposée, à la base, un perçage (8a) d'amenée de gaz sous pression, une introduction (10) de liquide à nébuliser étant formée dans le corps de manière communiquante avec le perçage (8a), l'introduction (10) étant raccordée, par l'intermédiaire d'une conduite, à une réserve de liquide à nébuliser, ledit perçage (8a) étant raccordé, par l'intermédiaire d'une conduite, à une source de gaz sous pression, le liquide étant aspiré dans la chambre (5) de mélange et de fractionnement sous l'effet de la dépression crée par le passage dans la chambre (5) du flux de gaz depuis le perçage (8a) vers l'ajutage de sortie (6) et étant finement fractionné et mélangé au flux de gaz sous pression sous l'effet de turbulences régnant dans la chambre (5), laquelle présente un épanouissement entre le perçage (8a) et l'ajutage de sortie (6), **caractérisé en ce que** :
- l'épanouissement est formé d'une surface de révolution,
- la largeur de l'introduction (10) de liquide, à son intersection avec la chambre (5), mesurée de manière perpendiculaire à l'axe du perçage (8a) est supérieure à la largeur du perçage (8a), mesurée de la même manière,
- l'épanouissement et le perçage (8a) d'amenée de gaz sont coaxiaux,
- l'introduction (10) de liquide est radiale au sens d'écoulement de gaz en sortie du perçage (8a),
- un premier ajutage (9) est disposé à l'intersection de l'introduction (10) de liquide et de la chambre (5),
- un autre ajutage (9'), ci-après nommé troisième ajustage, est formé en extrémité aval du premier perçage (8a) au niveau de son intersection avec l'introduction (10) de liquide,
- le premier ajutage (9) et le troisième ajutage (9') sont en extrémité de l'introduction (10) de liquide,
- le premier ajutage (9) et le troisième ajutage (9') sont écarté l'un de l'autre,
- le premier ajutage (9) et le troisième ajutage (9') sont formés par des arêtes vives,
de sorte que le jet conique formé en sortie de la tête est conique et est constitué de fines particules au centre et de grosses particules en périphérie.

2. Tête de nébulisation selon la revendication 1 **caractérisée en ce que** :
- l'introduction (10) est constituée par au moins un perçage cylindrique radial terminé par un second ajutage formé et dans la chambre (5) de mélange et de fractionnement et dans le perçage (8),
- l'axe géométrique longitudinal de symétrie de la zone du second perçage, située immédiatement en amont du second ajutage (11) est contenu dans le plan géométrique contenant le premier ajutage (9) lequel est délimité par une arête vive formée par l'intersection de l'épanouissement avec la zone amont du premier perçage,
- l'axe longitudinal de symétrie de la zone du second perçage (10) située immédiatement en amont du second ajutage (11) est sécant et perpendiculaire à l'axe de symétrie de l'épanouissement.

3. Tête de nébulisation (1) selon la revendication 2, **caractérisée en ce qu'**elle présente plusieurs seconds ajutages (11) répartis autour de l'axe de symétrie de la chambre.

4. Tête de nébulisation (1) selon la revendication 2 ou la revendication 3, **caractérisée en ce que** la zone (8a) du premier perçage, située immédiatement en amont du premier ajutage et la zone du second perçage située immédiatement en amont du second ajutage, sont toutes deux cylindriques.

5. Tête de nébulisation (1) selon la revendication 1, **caractérisé en ce que** l'introduction (10) de liquide s'étend entre et autour du premier ajutage (9) et du troisième ajutage (9').

6. Tête de nébulisation (1) selon la revendication 5 **caractérisée en ce que** la tête de nébulisation est constituée d'un corps en deux parties assemblées l'une à l'autre de manière démontable, la chambre (6) de mélange et de fractionnement ainsi que l'introduction (10) étant formées dans la première partie de corps, tandis que la seconde partie du corps est équipée du premier perçage (8) et des conduites de raccordement du perçage (8) et de l'introduction (10) à la source de gaz sous pression et à la réserve de liquide à nébuliser.

7. Tête de nébulisation (1) selon la revendication 6, **caractérisée en ce que** le perçage 8 est formé dans une buse amovible (32) introduite dans un logement de la seconde partie de corps.

8. Tête de nébulisation (1) selon l'une quelconque des revendications précédentes, **caractérisée en ce que** l'épanouissement s'étend depuis le premier ajutage (9) jusqu'à l'ajutage de sortie (6).

9. Tête de nébulisation (1) selon l'une quelconque des revendications précédentes, **caractérisée en ce que** l'épanouissement se présente sous la forme d'un tronc de cône dont l'angle au sommet est compris dans une fourchette de 10 à 90 degrés.

10. Tête de nébulisation (1) selon la revendication 9, **caractérisée en ce que** l'angle au sommet est égal à 60 degrés.

11. Tête de nébulisation (1) selon l'une quelconque des revendications précédentes, **caractérisée en ce que** l'ajutage de sortie (6) est contenu dans un plan géométrique normal à l'axe longitudinal de symétrie de la chambre (5) de mélange et fractionnement et est formé par une arête vive.

12. Tête de nébulisation (1) selon l'une quelconque des revendications précédentes, **caractérisée en ce que** le corps, autour de l'ajutage de sortie présente une surface en pente (7).

13. Tête de nébulisation (1) selon l'une quelconque des revendications précédentes, **caractérisée en ce qu'**au-dessus de l'ajutage de sortie (6), à écartement de ce dernier, est monté un déflecteur (14) ce déflecteur étant normal à l'axe de symétrie de la chambre (5) et centré par rapport à ce dernier, ledit déflecteur (14) étant destiné à rabattre le jet vers l'ajutage de sortie (6).

14. Tête de nébulisation selon la revendication 13, **caractérisée en ce que** le déflecteur (14) est écarté de l'ajutage de sortie d'une valeur supérieure à 5 µ m.

15. Tête de nébulisation (1) selon l'une quelconque des revendications précédentes, **caractérisée en ce qu'**elle est équipée d'au moins un perçage (2a) prévu pour l'aspiration des écoulements résiduels de liquide formés par les grosses particules, ce second perçage (2a) débouchant d'une part dans la chambre de mélange et de fractionnement (5) et d'autre part sur l'une des faces du corps de la tête ou il forme à ce niveau un orifice d'aspiration des écoulements résiduels de liquide nébulisé.

16. Appareil de nébulisation, **caractérisée en ce qu'**il est équipé d'au moins une tête de nébulisation (1) selon l'une quelconque des revendications précédentes.

17. Appareil de nébulisation selon la revendication 16, **caractérisé en ce qu'**il comprend un corps creux (16) à la base duquel est monté au moins une tête de nébulisation (1) en relation d'une part avec une source de gaz comprimé par l'intermédiaire d'une conduite d'alimentation (17) et d'autre part avec un réservoir (18) de liquide à nébuliser, monté sous le corps, la tête de nébulisation (1), par son ajutage de sortie (6) est en communication avec une chambre de détente (20) pratiquée dans le corps (16), coaxialement à la chambre de mélange et de fractionnement (5) de la tête de nébulisation (1), la face interne de ladite chambre recevant les grosses particules émises par le jet et captant ces dernières, lesquelles sous l'effet de leur poids s'écoulent vers le bas sur ladite face.

18. Appareil de nébulisation (15) selon la revendication 17, **caractérisé en ce que** la face interne de la chambre de détente et de captage des grosses particules est lisse ou comporte un ou plusieurs creux (21) et saillies (22) annulaires.

19. Appareil de nébulisation (15) selon la revendication 17 ou la revendication 18, **caractérisé en ce que** la chambre de détente (20) est prolongée par au moins une zone d'accélération du mouvement du brouillard de particules afin d'augmenter la vitesse de ce dernier et éviter tout dépôt de particules fines, ladite zone étant en relation avec un conduit de sortie du brouillard ou de l'aérosol formé.

20. Appareil de nébulisation (15) selon la revendication 19, **caractérisé en ce que** la zone (23) d'accélération du mouvement du brouillard est en relation avec la chambre de détente (20) par un passage (24) de section plus faible que la section de ladite chambre.

21. Appareil de nébulisation (15) selon la revendication 20, **caractérisé en ce que** la zone d'accélération (20) comprend au moins deux cloisons (25) s'étendant transversalement dans le corps creux (16) et montées à écartement l'une de l'autre, chaque cloison (25) comportant un passage (24) de section réduite constitué par un perçage traversant se présentant sous forme de lumière oblongue, les cloisons étant positionnées l'une par rapport à l'autre en sorte que la lumière traversante de l'une soit décalée angulairement par rapport à la lumière traversante de l'autre.

22. Appareil de nébulisation (15) selon l'une quelconque des revendications 17 à 21, **caractérisé en ce que** la chambre de détente (20) à l'opposée de la tête de nébulisation (1) est limitée par une paroi transversale (21) perpendiculaire à l'axe géométrique de la chambre de fractionnement de ladite tête, cette paroi en son centre comportant un orifice traversant (22) de sortie de la nébulisation.

23. Appareil de nébulisation (15) selon la revendication 22, **caractérisé en ce que** la face externe à la chambre de nébulisation, de la paroi (21) est convexe et que la paroi en périphérie comporte des échancrures.

24. Appareil de nébulisation (15) selon la revendication 17 ou la revendication 18, **caractérisé en ce que** la chambre de détente (20), à l'opposé de la tête de nébulisation (1), comporte une paroi d'obturation (27) pleine et que la paroi du réservoir (18) au-dessus du niveau maximal de liquide est équipée d'un embout de sortie (29) du brouillard ou de l'aérosol.

25. Appareil de nébulisation (15) selon l'une quelconque des revendications 17 à 24, **caractérisé en ce que** le corps (16), entre le réservoir (18) et la chambre de détente (20), est doté d'une cloison transversale de séparation (19) portant la tête de nébulisation (1).

26. Appareil de nébulisation (15) selon l'une quelconque des revendications 17 à 25, **caractérisé par** un moyen d'évacuation vers le réservoir, des écoulements résiduels de liquide formés par les grosses particules dans la chambre de détente (20).

27. Appareil de nébulisation (15) selon les revendications 25 et 26 prises ensembles, **caractérisé en ce que** le moyen d'évacuation des écoulements résiduels vers le réservoir (18) traverse de part en part la cloison de séparation (19), ce moyen, par son extrémité inférieure, étant situé en dessous du niveau minimal de liquide dans le réservoir (18).

28. Appareil de nébulisation (15) selon la revendication 27, **caractérisé en ce que** le moyen d'évacuation vers le réservoir, des écoulements résiduels de liquide formés par les grosses particules, est constitué par au moins un tube (30) engagé dans un perçage pratiqué dans la cloison de séparation (19), ladite cloison (19) et ledit tube (30) assurant de plus la séparation étanche entre le volume du réservoir (18) situé au-dessus du niveau de liquide et la chambre de détente (20).

29. Appareil de nébulisation (15) selon les revendications 24 et 25 prises ensembles, **caractérisé en ce que** la cloison transversale de séparation (19) est pourvue d'une ouverture (28) assurant la communication entre la chambre de détente (20) et le volume du réservoir situé au-dessus du niveau de liquide.

30. Appareil de nébulisation (15), selon l'une quelconque des revendications 17 à 21 **caractérisé en ce que** :
- il est monté de manière coulissante dans le réservoir (18) à la manière d'un piston,
- la partie inférieure du corps de l'appareil est équipé d'une cloison de séparation (19) agencée en piston,
- l'alimentation en air comprimé est effectuée par une canule rigide (49), en communication avec le perçage (8) du corps de la tête de nébulisation (1) par l'intermédiaire d'un perçage axial traversant, pratiqué dans la cloison de séparation (19),
- la canule (41) s'étend dans le réservoir (18) selon la direction de coulissement du corps de l'appareil, et est engagée en coulissement dans un alésage pratiqué dans la paroi de fond du réservoir (18),
- la canule (41) traverse de part en part le fond du réservoir (18), et est connectée à l'extérieur du réservoir (18), par une conduite souple, à une source de distribution d'air sous pression,
- il est associé à une pompe (50), dont l'orifice d'aspiration de liquide est connecté par une conduite appropriée, au volume interne d'une réserve de liquide (51) et dont l'orifice de refoulement, par l'intermédiaire d'une conduite appropriée, est connecté au volume interne du réservoir (18),
- la pompe (50) est actionnée par un moteur électrique, piloté par un circuit d'automatisme et de puissance comprenant notamment à l'extérieur du réservoir (18), deux capteurs de niveau (52), (53), distants l'un de l'autre et disposés tous deux sur le trajet d'un plot d'actionnement (54) porté par la canule (49).

31. Appareil de nébulisation (15) selon l'une quelconque des revendications 16 à 30, **caractérisé en ce qu'**il est équipé de plusieurs têtes de nébulisation (1) réparties à intervalles réguliers ou irréguliers selon un cercle.

32. Appareil de nébulisation selon l'une quelconque des revendications 16 à 31, **caractérisé en ce qu'**il est équipé de plusieurs têtes de nébulisation (1) réparties à intervalles réguliers ou irréguliers selon une droite.

33. Appareil de nébulisation selon l'une quelconque des revendications 16 à 32 **caractérisé par** des moyens pour maintenir constant le niveau de liquide dans le réservoir (18).

## Patentansprüche

1. Vernebelungskopf (1) mit einem Körper, in welchem eine offene Misch-und Fraktionierkammer (5) gebildet ist, die mit einer Austrittsdüse (6) und an der gegenüberliegenden Basis mit einer Bohrung (8a) für die Zuleitung von Druckgas versehen ist, wobei ein Einlaß (10) der zu vernebelnden Flüssigkeit im Körper so gebildet ist, daß er mit der Bohrung (8a) in Verbindung steht, der Einlaß (10) mittels einer Leitung mit einem Vorrat der zu vernebelnden Flüssigkeit verbunden ist, die Bohrung (8a) mittels einer Leitung mit einer Druckgasquelle verbunden ist, die Flüssigkeit unter der Wirkung des Unterdrucks in die Misch- und Fraktionierkammer (5) angesaugt wird, der durch den Durchtritt des Gasstroms von der Bohrung (8a) in die Kammer (5) zur Auslaßdüse (6) erzeugt und unter der Wirkung von in der Kammer (5) herrschenden Wirbeln zerteilt und mit dem Druckgasstrom vermischt wird, wobei die Kammer (5) zwischen der Bohrung (8a) und der Auslaßdüse (6) eine Erweiterung aufweist, **dadurch gekennzeichnet, daß**
- die Erweiterung von einer Rotationsfläche gebildet ist,
- die Breite des Flüssigkeitseinlasses (10) an ihrer Schnittstelle mit der Kammer (5) senkrecht zur Achse der Bohrung (8a) gemessen größer als die in gleicher Weise gemessene Breite der Bohrung (8a) ist,
- die Erweiterung und die Gaszuleitungsbohrung (8a) koaxial sind,
- der Flüssigkeitseinlaß (10) radial zur Strömungsrichtung des aus der Bohrung (8a) austretenden Gases ist;
- eine erste Düse (9) an der Schnittstelle des Flüssigkeitseinlasses (10) und der Kammer (5) angeordnet ist,
- eine weitere Düse (9'), hiernach als dritte Düse bezeichnet, am stromabwärtsliegenden Ende der ersten Bohrung (8a), auf der Höhe von deren Schnitt mit dem Flüssigkeitseinlaß (10) ausgebildet ist,
- die erste Düse (9) und die dritte Düse (9') am Ende der Flüssigkeitseinleitung (10) sind,
- die erste Düse (9) und die dritte Düse (9') voneinander entfernt sind,
- die erste Düse (9) und die dritte Düse (9') von scharfen Kanten gebildet sind, so daß der am Ausgang des Kopfes gebildete kegelförmige Strahl kegelförmig ist und aus feinen Teilchen in der Mitte und groben Teilchen am Rand besteht.

2. Vernebelungskopf nach Anspruch 1, **dadurch gekennzeichnet, daß** der Einlaß (10) aus mindestens einer radialen zylindrischen Bohrung besteht, die in einer zweiten Düse endet, die sowohl in der Misch- und Fraktionierkammer (5) wie in der Bohrung (8) ausgebildet ist,
- die geometrische Symmetrie-Längsachse der Zone der zweiten Bohrung unmittelbar stromaufwärts von der zweiten Düse (11) und in der geometrischen Ebene liegt, welche die erste Düse (9) enthält, welche durch eine scharfe Kante begrenzt ist, die vom Schnitt der Erweiterung mit der stromaufwärts von der ersten Bohrung liegenden Zone gebildet ist,
- die Symmetrie-Längsachse der Zone der zweiten Bohrung (10), die unmittelbar stromaufwärts von der zweiten Düse (11) liegt, die Symmetrieachse der Erweiterung schneidet und dazu senkrecht ist.

3. Vernebelungskopf (1) nach Anspruch 2, **dadurch gekennzeichnet, daß** er mehrere rings um die Symmetrieachse der Kammer verteilte zweite Düsen (11) aufweist.

4. Vernebelungskopf (1) nach Anspruch 2 oder Anspruch 3, **dadurch gekennzeichnet, daß** die unmittelbar stromaufwärts von der ersten Düse liegende Zone (8a) der ersten Bohrung und die unmittelbar stromaufwärts von der zweiten Düse liegende Zone der zweiten Bohrung beide zylindrisch sind.

5. Vernebelungskopf (1) nach Anspruch 1, **dadurch gekennzeichnet, daß** der Flüssigkeitseinlaß (10) sich zwischen und rings um die erste Düse (9) und dritte Düse (9') erstreckt.

6. Vernebelungskopf (1) nach Anspruch 5, **dadurch gekennzeichnet, daß** der Vernebelungskopf aus einem Körper besteht, der aus zwei abnehmbar miteinander vereinigten Teilen besteht, wobei die Misch- und Fraktionierkammer (6) sowie der Einlaß (10) im ersten Teil des Körpers gebildet sind während der zweite Teil des Körpers mit der ersten Bohrung (8) und Leitungen für die Verbindung der Bohrung (8) und des Einlasses (10) mit der Druckgasquelle und dem Vorrat der zu vernebelnden Flüssigkeit ausgerüstet sind.

7. Vernebelungskopf (1) nach Anspruch 6, **dadurch gekennzeichnet, daß** die Bohrung (8) in einer abnehmbaren Düse (32) gebildet ist, die in einen Sitz des zweiten Teils des Körpers eingerührt ist.

8. Vernebelungskopf (1) nach einem der vorangehenden Ansprüche, **dadurch gekennzeichnet, daß** die Erweiterung sich von der ersten Düse (9) bis zur Auslaßdüse (6) erstreckt.

9. Vernebelungskopf (1) nach einem der vorangehenden Ansprüche, **dadurch gekennzeichnet, daß** die Erweiterung die Form eines Kegelstumpfes hat, dessen spitzer Winkel in einem Bereich von 10 bis 90 Grad liegt.

10. Vernebelungskopf (1) nach Anspruch 9, **dadurch gekennzeichnet, daß** der spitze Winkel 60 Grad beträgt.

11. Vernebelungskopf (1) nach einem der vorangehenden Ansprüche, **dadurch gekennzeichnet, daß** die Auslaßdüse (6) in einer geometrischen Ebene liegt, die normal zur Symmetrielängsachse der Misch- und Fraktionierkammer (5) ist, und von einer scharfen Kante gebildet ist.

12. Vernebelungskopf (1) nach einem der vorangehenden Ansprüche, **dadurch gekennzeichnet, daß** der Körper rings um die Auslaßdüse eine Schrägfläche (7) aufweist.

13. Vernebelungskopf (1) nach einem der vorangehenden Ansprüche, **dadurch gekennzeichnet, daß** oberhalb der Auslaßdüse (6) in einem Abstand von dieser ein Deflektor (14) montiert ist, der zur Symmetrieachse der Kammer (5) normal und zu dieser zentriert ist, wobei der Deflektor (14) dazu dient, den Strahl zur Auslaßdüse (6) zurückzuleiten.

14. Vernebelungskopf nach Anspruch 13, **dadurch gekennzeichnet, daß** der Deflektor (14) um mehr als 5 µm von der Auslaßdüse entfernt ist.

15. Vernebelungskopf (1) nach einem der vorangehenden Ansprüche, **dadurch gekennzeichnet, daß** er mit mindestens einer Bohrung (2a) versehen ist, die zum Ansaugen von Restabläufen von Flüssigkeit dient, die von den großen Teilchen gebildet sind, wobei diese zweite Bohrung (2a) einerseits in der Misch- und Fraktionierkammer (5) und andererseits an einer der Flächen des Körpers des Kopfes mündet, wo er auf dieser Höhe eine Ansaugöffnung für Restabläufe der vernebelten Flüssigkeit bildet.

16. Vernebelungsvorrichtung **dadurch gekennzeichnet, daß** sie mit mindestens einem Vernebelungskopf (1) nach einem der vorangehenden Ansprüche ausgerüstet ist.

17. Vernebelungsvorrichtung nach Anspruch 16, **dadurch gekennzeichnet, daß** sie einen hohlen Körper (16) aufweist, an dessen Basis mindestens ein Vernebelungskopf (1) montiert ist, der einerseits durch eine Versorgungsleitung (17) mit einer Druckgasquelle und andererseits mit einem Vorratsbehälter (18) für die zu vernebelnde Flüssigkeit, der unter dem Körper montiert ist, in Beziehung ist, wobei der Vernebelungskopf (1) durch seine Auslaßdüse (6) mit einer im Körper (16) ausgebildeten Entspannungskammer (20) in Verbindung ist, die koaxial zur Misch- und Fraktionierkammer (5) des Vernebelungskopfes (1) ist, wobei die Innenseite der Kammer die großen Teilchen aufnimmt, die vom Strahl ausgesandt werden, und diese abfängt, die unter der Wirkung ihres Gewichts auf dieser Innenseite ablaufen.

18. Vernebelungsvorrichtung (15) nach Anspruch 17, **dadurch gekennzeichnet, daß** die Innenseite der zur Entspannung und zum Auffangen großer Teilchen dienenden Kammer glatt ist oder ein oder mehrere hohle Ringe (21) und vorspringende Ringe (22) aufweist.

19. Vernebelungsvorrichtung (15) nach Anspruch 17 oder Anspruch 18, **dadurch gekennzeichnet, daß** die Entspannungskammer (20) durch mindestens eine Zone der Beschleunigung der Bewegung des Nebels von Teilchen verlängert ist, um dessen Geschwindigkeit zu erhöhen und jede Abscheidung feiner Teilchen zu vermeiden, wobei diese Zonen in Beziehung stehen zu einer Ausgangsleitung des gebildeten Nebels oder Aerosols.

20. Vernebelungsvorrichtung (15) nach Anspruch 19, **dadurch gekennzeichnet, daß** die Zone (23) zur Beschleunigung der Bewegung des Nebels durch einen Durchlaß (24) in Beziehung steht zur Entspannungskammer (20), wobei der Durchlaß (24) einen geringeren Querschnitt als der Querschnitt der Kammer hat.

21. Vernebelungsvorrichtung (15) nach Anspruch 20, **dadurch gekennzeichnet, daß** die Zone der Beschleunigung (20) mindestens zwei Trennwände (25) aufweist, die sich quer im hohlen Körper (16) erstrecken und in einem Abstand voneinander angeordnet sind, wobei jede Trennwand (25) einen Durchlaß (24) von verringertem Querschnitt aufweist, der gebildet ist durch eine Durchgangsbohrung, welche die Form eines länglichen Schlitzes hat, wobei die Trennwände zueinander so angeordnet sind, daß der Durchgangsschlitz der einen in einem Winkel bezüglich des Durchgangsschlitzes der anderen versetzt ist.

22. Vernebelungsvorrichtung (15) nach einem der Ansprüche 17 bis 21, **dadurch gekennzeichnet, daß** die Entspannungskammer (20) gegenüber dem Vernebelungskopf (1) durch eine Querwand (21) begrenzt ist, die senkrecht zur geometrischen Achse der Fraktionierkammer des Kopfes verläuft, wobei diese Wand in ihrer Mitte eine Durchgangsöffnung (22) für den Austritt des erzeugten Nebels aufweist.

23. Vernebelungsvorrichtung (15) nach Anspruch 22, **dadurch gekennzeichnet, daß** die hinsichtlich der Vernebelungskammer außen liegende Seite der Wand (21) konvex ist und die Wand am Rand Einbuchtungen aufweist.

24. Vernebelungsvorrichtung (15) nach Anspruch 17 oder Anspruch 18, **dadurch gekennzeichnet, daß** die Entspannungskammer (20) gegenüber dem Vernebelungskopf (1) eine voll ausgebildete Verschlußwand (27) aufweist und daß die Wand des Vorratsbehälters (18) oberhalb des maximalen Flüssigkeitsniveaus mit einem Austrittsstutzen (29) für den Nebel oder das Aerosol ausgerüstet ist.

25. Vernebelungsvorrichtung (15) nach einem der Ansprüche 17 bis 24, **dadurch gekennzeichnet, daß** der Körper (16) zwischen dem Vorratsbehälter (18) und der Entspannungskammer (20) mit einer quer verlaufenden Trennwand (19) ausgerüstet ist, die den Vernebelungskopf (1) trägt.

26. Vernebelungsvorrichtung (15) nach einem der Ansprüche 17 bis 25, **gekennzeichnet durch** eine Vorrichtung zum Abführen von Flüssigkeitsrestabläufen, die von den großen Teilchen in der Entspannungskammer (20) gebildet sind, zum Vorratsbehälter.

27. Vernebelungsvorrichtung (15) nach den Ansprüchen 25 und 26 zusammengenommen, **dadurch gekennzeichnet, daß** die Vorrichtung zur Abführung von Restabläufen zum Vorratsbehälter (18) die Trennwand (19) von der einen zur anderen Seite durchsetzt, wobei diese Vorrichtung mit ihrem unteren Ende unterhalb des Mindestniveaus der Flüssigkeit im Vorratsbehälter (18) angeordnet ist.

28. Vernebelungsvorrichtung (15) nach Anspruch 27, **dadurch gekennzeichnet, daß** die Vorrichtung zur Abführung von durch die großen Teilchen gebildeten Flüssigkeitsrestabläufen zum Vorratsbehälter aus mindestens einem Rohr (30) besteht, das in eine Bohrung eingesetzt ist, die in der Trennwand (19) ausgebildet ist, wobei die Trennwand (19) und das Rohr (30) außerdem für die dichte Trennung zwischen dem Volumen des Vorratsbehälters (18) oberhalb des Niveaus der Flüssigkeit und der Entspannungskammer (20) sorgen.

29. Vernebelungsvorrichtung (15) nach den Ansprüchen 24 und 25 zusammengenommen, **dadurch gekennzeichnet, daß** die quer verlaufende Trennwand (19) mit einer Öffnung (28) versehen ist, welche die Verbindung zwischen der Entspannungskammer (20) und dem oberhalb des Niveaus der Flüssigkeit liegenden Volumen des Vorratsbehälters herstellt.

30. Vernebelungsvorrichtung (15) nach einem der Ansprüche 17 bis 21, **dadurch gekennzeichnet, daß**
- sie nach Art eines Kolbens verschiebbar im Vorratsbehälter (18) montiert ist,
- der untere Teil des Körpers der Vorrichtung mit einer als Kolben ausgebildeten Trennwand (19) ausgerüstet ist,
- die Versorgung mit Druckluft durch eine starre Kanüle (49) erfolgt, die mittels einer in der Trennwand (19) ausgebildeten, sie axial durchstoßenden Bohrung mit der Bohrung (8) des Körpers des Vernebelungskopfes (1) in Verbindung steht,
- die Kanüle (41) sich im Vorratsbehälter (18) in der Verschiebungsrichtung des Körpers der Vorrichtung erstreckt und verschiebbar in eine in der Wand des Bodens des Vorratsbehälters (18) ausgebildete Bohrung eingesetzt ist;
- die Kanüle (41) den Boden des Vorratsbehälters (18) von der einen zur anderen Seite durchsetzt und außerhalb des Vorratsbehälters (18) durch eine nachgiebige Leitung mit einer Druckluftquelle verbunden ist,
- sie mit einer Pumpe (50) ausgerüstet ist, deren Flüssigkeitsansaugöffnung durch eine entsprechende Leitung mit dem Innenvolumen eines Flüssigkeitsvorrats (51) verbunden ist und deren Abgabeöffnung mittels einer geeigneten Leitung mit dem Innenvolumen des Vorratsbehälters (18) verbunden ist,
- die Pumpe (50) durch einen Elektromotor betätigt ist, der durch einen Automatik- und Leistungskreis gesteuert ist, der besonders außerhalb des Vorratsbehälters (18) zwei Niveaufühler (52, 53) aufweist, die voneinander entfernt sind und alle beide im Weg eines von der Kanüle (49) getragenen Betätigungsklotzes (54) angeordnet sind.

31. Vernebelungsvorrichtung (15) nach einem der Ansprüche 16 bis 30, **dadurch gekennzeichnet, daß** sie mit mehreren Vernebelungsköpfen (1) ausgerüstet ist, die in gleichmäßigen oder ungleichmäßigen Abständen gemäß einen Kreis verteilt sind.

32. Vernebelungsvorrichtung nach einem der Ansprüche 16 bis 31, **dadurch gekennzeichnet, daß** sie mit mehreren Vernebelungsköpfen (1) ausgerüstet ist, die in regelmäßigen oder unregelmäßigen Abständen gemäß einer Geraden verteilt sind.

33. Vernebelungsvorrichtung nach einem der Ansprüche 16 bis 32, **gekennzeichnet durch** Mittel, um das Flüssigkeitsniveau im Vorratsbehälter (18) konstant zu halten.

## Claims

1. Spraying head (1) comprising a body in which is formed an open mixing and fractioning chamber (5) fitted with an outlet nozzle (6) and, oppositely, at the base, a pressurized gas inlet hole (8a), an inlet (10) for liquid to be sprayed being created in the body such that it communicates with the hole (8a), the inlet (10) being connected, by means of a duct, to a reservoir of liquid to be sprayed, the aforementioned hole (8a) being connected, by means of a duct, to a pressurized gas source, the liquid being drawn into the mixing and fractioning chamber (5) under the effect of negative pressure created by the passage of a gas flow in the chamber (5) from the hole (8a) to the outlet nozzle (6) and being finely fractioned and mixed with the pressurized gas flow under the effect of turbulence present in the chamber (5), which features a flare between the hole (8a) and the outlet nozzle (6), **characterised in that**:
- the flare is formed by a surface of revolution,
- the width of the liquid inlet (10), at its intersection with the chamber (5), measured perpendicularly to the hole (8a) axis is greater than the width of the hole (8a), measured similarly,
- the flare and the gas inlet hole (8a) are coaxial,
- the liquid inlet (10) is radial to the gas flow direction at the hole (8a) outlet,
- a first nozzle (9) is placed at the intersection of the liquid inlet (10) and the chamber (5),
- another nozzle (9'), hereinafter referred to as the third nozzle, is formed at the downstream endpoint of the first hole (8a) at the point where the hole (8a) intersects with the liquid inlet (10),
- the first nozzle (9) and the third nozzle (9') are at the endpoint of the liquid inlet (10),
- the first nozzle (9) and the third nozzle (9') are placed at a distance from each other,
- the first nozzle (9) and the third nozzle (9') are formed by sharp edges,
such that the conical spray formed at the head outlet is conical and comprised of fine particles at the centre and large particles at the periphery.

2. Spraying head according to claim 1 **characterised in that**:
- the inlet (10) is comprised of at least one radial cylindrical hole ending in a second nozzle formed in both the mixing and fractioning chamber (5) and in the hole (8),
- the geometric longitudinal axis of symmetry of the second hole zone, located immediately upstream from the second nozzle (11) is contained in the geometric plane containing the first nozzle (9) which is delimited by a sharp edge formed by the intersection of the flare with the upstream zone of the first hole,
- the longitudinal axis of symmetry of the second hole (10) zone located immediately upstream from the second nozzle (11) is secant and perpendicular to the axis of symmetry of the flare.

3. Spraying head (1) according to claim 2, **characterised in that** it features several second nozzles (11) distributed around the chamber's axis of symmetry.

4. Spraying head (1) according to claim 2 or claim 3, **characterised in that** the zone (8a) of the first hole, located immediately upstream from the first nozzle and the zone of the second hole located immediately upstream from the second nozzle are both cylindrical.

5. Spraying head (1) according to claim 1, **characterised in that** the liquid inlet (10) extends between and around the first nozzle (9) and the third nozzle (9').

6. Spraying head (1) according to claim 5, **characterised in that** the spraying head is comprised of a body in two sections attached one to the other such that they may be disassembled, the mixing and fractioning chamber (6) as well as the inlet (10) being formed in the first section of the body, while the second section of the body contains the first hole (8) and the connection ducts of the hole (8) and the inlet (10) to the pressurized gas source and the reservoir of liquid to be sprayed.

7. Spraying head (1) according to claim 6, **characterized in that** the hole 8 is made in a removable nozzle (32) introduced into a housing of the second section of the body.

8. Spraying head (1) according to any one of the aforementioned claims, **characterised in that** the flare extends from the first nozzle (9) up to the outlet nozzle (6).

9. Spraying head (1) according to any one of the aforementioned claims, **characterised in that** the flare has the appearance of a truncated cone section the apex angle of which is included in a range of 10 to 90 degrees.

10. Spraying head (1) according to claim 9, **characterised in that** the apex angle is equal to 60 degrees.

11. Spraying head (1) according to any one of the aforementioned claims, **characterised in that** the outlet nozzle (6) is contained within a geometric plane perpendicular to the longitudinal axis of symmetry of the mixing and fractioning chamber (5) and is formed by a sharp edge.

12. Spraying head (1) according to any one of the aforementioned claims, **characterised in that** the body, around the outlet nozzle features a sloped surface (7).

13. Spraying head (1) according to any one of the aforementioned claims, **characterised in that** above outlet nozzle (6), at a distance from the latter, is mounted a deflector (14), said deflector being perpendicular to the axis of symmetry of the chamber (5) and centred with respect to the latter, said deflector (14) being intended to knock the spray towards the outlet nozzle (6).

14. Spraying head according to claim 13, **characterised in that** the deflector (14) is separated from the outlet nozzle by a value greater than 5 µm.

15. Spraying head (1) according to any one of the aforementioned claims, **characterised in that** it features at least one hole (2a) provided to draw up residual liquid flow formed by large particles, this second hole (2a) coming out on the one hand in the mixing and fractioning chamber (5) and, on the other, on one of the surfaces of the head body where it forms at this point an opening drawing up residual flow of sprayed liquid.

16. Spraying device, **characterised in that** it is fitted with at least one spraying head (1) according to any one of the aforementioned claims.

17. Spraying device according to claim 16, **characterised in that** it comprises a hollow body (16) at the base of which is mounted at least one spraying head (1) connected on the one hand to a compressed gas source by means of a supply duct (17) and the other with a reservoir (18) of liquid to be sprayed, mounted under the body, the spraying head (1), by means of its outlet nozzle (6) communicates with an expansion chamber (20) made in the body (16), coaxially to the mixing and fractioning chamber (5) of the spraying head (1), the internal surface of the aforementioned chamber receiving the large particles issued by the spray and capturing the latter, which flow downwards under gravity on the aforementioned surface.

18. Spraying device (15) according to claim 17, **characterised in that** the internal surface of the expansion and large particle capturing chamber is smooth or features one or more ring shaped protrusions (22) and hollows (21).

19. Spraying device (15) according to claim 17 or claim 18, **characterised in that** the expansion chamber (20) is prolonged by at least one particle mist movement acceleration zone in order to increase the speed of the latter and avoid any deposition of fine particles, the aforementioned zone being connected to a mist outlet or formed aerosol duct.

20. Spraying device (15) according to claim 19, **characterised in that** the mist movement acceleration zone (23) communicates with the expansion chamber (20) by a passage (24) with a cross-section smaller than the cross-section of the aforementioned chamber.

21. Spraying device (15) according to claim 20, **characterised in that** the acceleration zone (20) comprises at least two partitions (25) extending transversely within the hollow body (16) and mounted at a distance from each other, each partition (25) bearing a passage (24) of reduced cross-section constituted by a through hole that has the appearance of an elongated aperture, partitions being positioned with respect to each other such that the through aperture of one is angularly offset with respect to the through aperture of the other.

22. Spraying device (15) according to any one of the claims 17 to 21, **characterised in that** the expansion chamber (20) opposite the spraying head (1) is limited by a transversal wall (21) perpendicular to the geometrical axis of the fractioning chamber of the aforementioned head, the centre of said wall bearing a spraying outlet through opening (22).

23. Spraying device (15) according to claim 22, **characterised in that** the external surface of the spraying chamber, of the wall (21) is convex and that the peripheral wall features notches.

24. Spraying device (15) according to claim 17 or claim 18, **characterised in that** the expansion chamber (20) opposite the spraying head (1) comprises a solid sealing wall (27) and that the reservoir wall (18) above the maximum liquid level is fitted with an aerosol or mist outlet end piece (29).

25. Spraying device (15) according to any one of the claims 17 to 24, **characterised in that** the body (16), between the reservoir (18) and the expansion chamber (20) is equipped with a transversal separation partition (19) bearing the spraying head (1).

26. Spraying device (15) according to any one of the claims 17 to 25, **characterised by** a means of evacuating residual liquid flows formed by large particles in the expansion chamber (20) towards the reservoir.

27. Spraying device (15) according to claims 25 and 26 taken together, **characterised in that** the means of evacuating residual flow towards the reservoir (18) crosses from one side of the separation partition (19) to the other, said means, by its lower extremity, being located below the minimum level of liquid in the reservoir (18).

28. Spraying device (15) according to claim 27, **characterised in that** the means of evacuating residual liquid flows formed by large particles towards the reservoir, comprises at least one tube (30) inserted in a hole made in the separation partition (19), said partition (19) and said tube (30) furthermore ensuring leak-tight separation between the reservoir volume (18) located above the liquid level and the expansion chamber (20).

29. Spraying device (15) according to claims 24 and 25 taken together, **characterised in that** the transversal separation partition (19) features an opening (28) ensuring communication between the expansion chamber (20) and the reservoir volume located above the liquid level.

30. Spraying device (15) according to any one of the claims 17 to 21 **characterised in that**:
- it is mounted slidingly in the reservoir (18) in a manner comparable to that of a piston,
- the lower section of the body of the device is fitted with a separation partition (19) laid out as a piston,
- compressed air is supplied by means of a rigid canula (49), communicating with the hole (8) of the spraying head (1) body by means of axial through hole, made in the separation partition (19),
- the canula (41) extends into the reservoir (18) along the sliding direction of the device body, and is slidingly engaged in a bored hole made in the bottom plate of the reservoir (18),
- the canula (41) crosses the bottom of the reservoir (18) from one side to the other, and is connected at the outside of the reservoir (18) by a flexible tube, to a pressurised air distribution source,
- it is connected to a pump (50), the liquid drawing opening of which is connected by means of a suitable duct, to an internal volume of a liquid reservoir (51) and whose discharge opening is connected to an internal volume of the reservoir (18), by means of a suitable duct,
- the pump (50) is actuated by an electrical motor, controlled by an automation and power system notably comprising outside the reservoir (18), two level sensors (52), (53), placed at a distance from each other and both arranged on the path of an actuating stud (54) carried by the canula (49).

31. Spraying device (15) according to any one of the claims 16 to 30, **characterised in that** it is fitted with several spraying heads (1) distributed at regular or irregular intervals around a circle.

32. Spraying device according to any one of the claims 16 to 31, **characterised in that** it is fitted with several spraying heads (1) distributed at regular or irregular intervals along a straight-line.

33. Spraying device according to any one of the claims 16 to 32 **characterised by** the means to maintain a constant liquid level in the reservoir (18).
